# EUROPEAN PATENT APPLICATION

(11) **EP 1 498 093 A2**
(43) Date of publication of application: **19.01.2005**
(21) Application number: 04014727.4
(22) Date of filing: 23.06.2004
(51) Int. Cl.: A61F 13/20

(54) **Tampon with apertured non-woven cover**

(30) Priority: 18.07.2003 GB 0316823; 19.07.2003 GB 0317190
(71) Applicant: Accantia (Holdings) Limited, Birmingham B8 3DZ (GB)
(72) Inventor: Perry, Lisa, Birmingham B8 3DZ (GB)
(74) Representative: Harrison Goddard Foote

(57) **Abstract**

The invention relates to a tampon comprising a generally elongated absorbent core of absorbent material and an outer cover comprising a non-woven textile material formed from bi-component fibres which can be secured or bonded to the absorbent core.

## Description

The present invention relates to a novel form of tampon and to methods of manufacture related thereto.

Tampons with non-woven covers are well known. The cover not only aids to reduce the amount of fibre loss but also aids smoother insertion and withdrawal properties of tampons. Non-woven coverstocks made from bi-component fibres are preferred for tampons. The reason is that the heat-sealing of the sheath of a fibre can be activated without destroying the basis of the fibre structure or core.

Johnson & Johnson describe a non-woven bi-component coverstock in European Patent No. EP 0 149 155. Others have practically used bi-component fibre structured non-wovens but using different sealing arrangements to the aforementioned Johnson & Johnson Patent.

International Patent Application No. WO 02/056810 describes a heterogeneous film wrapping element for an absorbent article. The apertured film generally comprises a continuous phase of a thermoplastic polymeric component and an immiscible, dispersed phase of a thermoplastic polymeric component, having a lower melting point. Thus, the wrapping is useful in applications involving heat sealing processes in absorbent articles.

Non-woven fabrics may incorporate multicomponent fibres having at least two different melting points. Then these fabrics may be adhered to an absorbent article, such as a tampon, as described in Friese, U.S. Patent No. 4,816,000. This is one example of heat sealing a wrapping element or cover.

It may also be desirable to heat seal an apertured film. While it may be possible to heat seal an apertured film that is formed of only one component, this is generally only in areas where it is acceptable or even desirable that the apertures in the film become closed. However, heat sealing an apertured film to an underlying fibrous structure in a manner that maintains open apertures in the heat sealing area is more complex, and it generally requires the use of a film material having at least two different components having at least two different melting point temperatures. An example of this is described in Thompson *et al,* U.S. Patent No. 5,342,334. In this example, the apertured film is formed from a co-extruded film having a higher melting point polymeric material on a first, non-bonded surface, and a lower melting point polymeric material on a second, heat-bondable surface.

Use of bi-component fibre structures provides a major benefit of product softness in non-wovens. Other single component fibres are naturally harsher than bi-component particularly those with a polyethylene sheath. Some polymers, e.g. polyethylene fibres, are soft, but the melting point may make processing difficult. Also, specific fibre characteristics may cause difficulties in, *inter alia,* processing.

Thus, according to the invention we provide a tampon comprising a generally elongated absorbent core of absorbent material, and an outer cover comprising a non-woven material formed from bi-component fibres which can be thermally bonded to the absorbent core.

Preferentially, the non-woven material is an apertured non-woven coverstock.

In an alternative aspect of the invention we provide a tampon comprising a generally elongated absorbent core of absorbent material and an outer cover comprising an apertured bi-component polymeric fibrous coverstock thermally bonded to the absorbent core.

Although it is preferred that the material of the coverstock comprises a generally bi-component fibre, it will be understood by one skilled in the art that multi-component fibres, e.g. tri-component fibre, etc, may also be utilised in the present invention.

The apertured non-woven coverstock provides a generally smooth land area on the outermost surface of the tampon for gentle, nondrying passage along the vaginal walls. The apertures in the non-woven coverstock allow fluid to pass through the cover and into the absorbent core, where the fluid is held to prevent leakage.

The tampon of the invention may be made by forming a generally elongated blank of absorbent material, attaching a length of apertured polymeric formed coverstock so that it covers at least part of the generally elongated surface of the blank, and then compressing the covered blank to form a tampon. A withdrawal cord or string may be attached to or securely looped around the blank or the covered blank to aid a user in withdrawing the tampon from a body cavity after use.

The tampon of the invention may comprise a digital tampon or an applicator tampon. However, it is preferred that the invention comprises a novel form of digital tampon since a non-woven cover can aid tampon insertion.

Apertured polymeric formed non-woven coverstocks are known and may be made by a number of methods which are known to those in the art, including hot air aperturing and water aperturing. Non-wovens may also be made using other conventionally known textile technologies and apertured using other means, such as, perforation by heat or a combination of heat and pressure. The preferred non-woven for the present invention may be made using thermally bonded technology, wherein fibres are bonded with one another by heat. By way of example only, this may be achieved by, e.g. air through bonding, calender rollers, point bonding means or any combination of the aforesaid.

A representative, non-limiting list of suitable polymers for the first thermoplastic polymeric component of the fibre (known as the core) includes polyolefins, such as polypropylene and polyethylene; polyolefin copolymers, such as ethylene-vinyl acetate ("EVA"), ethylene-propylene, ethylene-acrylates, and ethylene-acrylic acid and salts thereof; halogenated polymers; polyesters and polyester copolymers; polyamides and polyamide copolymers; polyurethanes and polyurethane copolymers; polystyrenes and polystyrene copolymers; and the like. Preferred first thermoplastic polymeric components include polyolefins, especially polypropylene.

The thermoplastic element provides localized areas for thermal bonding to adjacent elements. The second thermoplastic polymeric component of the fibre (known as the sheath) provides these properties, and it may also contribute to some of the mechanical properties, including coefficient of friction. A representative, non-limiting list of suitable polymers for the second thermoplastic polymeric component includes polyolefins, such as polypropylene; polyolefin copolymers, such as ethylene-vinyl acetate ("EVA"), ethylene-propylene, ethylene-acrylates, and ethylene-acrylic acid and salts thereof; halogenated polymers; polyesters and polyester copolymers; polyamides and polyamide copolymers; polyurethanes and polyurethane copolymers; polystyrenes and polystyrene copolymers; and the like. Examples of second thermoplastic polymeric components include polyethylene and its copolymers, especially linear low density polyethylene (LLDPE), low density polyethylene (LDPE) and EVA.

The first and second thermoplastic polymeric components should be selected to allow the desired properties of each material to be apparent.

Preferred fibres are polyethylene (PE) sheath and polypropylene (PP) core or PE sheath / Polyester (PET) core. However, as technology on fibres and fibre finishes is improving one can see single component fibres such as PE or PP being introduced as the basis for the non-woven. A polypropylene/polyethylene coverstock is the most preferred material.

The mass per unit area of the coverstock may vary and may be from 10 to 30 gsm, preferably 14 to 22 gsm, more preferably from 16 to 20 gsm, most preferably 18 gsm.

The coverstock, or the bi-component fibre of the coverstock, may be provided with a hydrophobic or a hydrophilic finish. However, it is preferred that a hydrophilic finish is provided.

The caliper of the coverstock may vary, but is preferably less than 0.6 mm, more preferably less than 0.5 mm, most preferably less than 0.45 mm. Thus, a preferred caliper is in the range of from 0.35 to 0.45 mm, preferably from 0.4 to 0.45 mm. A caliper of 0.41mm is especially preferred.

The tensile strength of the coverstock may vary, but is preferably from 5 to 15 N/50mm (MD - machine direction), preferably from 8 to 12 N/50mm (MD) and most preferably 10 N/50mm (MD).

The elongation at breaking point of the coverstock may vary, but is preferably less than 60% (MD), more preferably less than 50% (MD), most preferably less than 30% (MD) and especially less than 20% (MD). An elongation at breaking point of the coverstock of 10% (MD) is particularly preferred.

The absorbent structure may be any absorbent means that is capable of absorbing and/or retaining liquids (e.g., menses and/or urine). The absorbent structure can be manufactured in a wide variety of sizes and shapes and from a wide variety of liquid-absorbing materials. A representative, non-limiting list of useful materials includes cellulosic materials, such as rayon, cotton, wood pulp, creped cellulose wadding, tissue wraps and laminates, peat moss, and chemically stiffened, modified, or cross-linked cellulosic fibres; synthetic materials, such as polyester fibres, polyolefin fibres, absorbent foams, absorbent sponges, superabsorbent polymers, absorbent gelling materials; formed fibres, such as capillary channel fibres and multilimbed fibres; combinations of materials, such as synthetic fibres and wood pulp including coformed fibrous structures.

In addition, other components and further additives can be added to the absorbent structure in an amount that will not hinder obtaining the object of the present invention, including, without limitation, antioxidants, UV absorbers, lubricants, antiblock and slip agents, plasticizers, nucleating agents, antistatic agents, flame retardants, pigments, dyes and inorganic or organic fillers.

Moreover, such components may also be added to the coverstock. However, in an especially preferred embodiment of the present invention other materials may be added to the coverstock, such as, surfactants, antimicrobials, zeolites, medicaments and the like which might result in offering, for example, benefits of insertion, method of drug delivery, pH balancing properties etc.

By aperturing the non-woven, there is more free space and openness within the material structure. Such structures have a greater moisture vapour permeability and air permeability than closed structures.

Therefore, improvements are made to tampons and demonstrated in-vivo, whilst maintaining the key benefits of current tampons of insertion and withdrawal benefits. These benefits manifest in that the absorbent cotton / rayon fibre is under the surface material of lower coefficient of friction.

Tampons are used to varying degrees of absorption during in-vivo use and the viscosity of menstrual fluid and fragments of blood can remain on the non-woven surface. Aperturing the surface of the non-woven enables a more attractive pattern of absorbency with fluid transferring more directly into the absorbent core.

The size and number of the apertures in the coverstock are chosen to minimise the penetration of absorbent core materials through the coverstock. The presence of absorbent core materials on the surface of the tampon both increases friction against the vaginal wall upon insertion and contributes to desiccation of the vaginal wall during use, leading to removal discomfort. The absence of absorbent core materials on the surface of the tampon helps to maintain the ease of insertion and removal. The size and/or pattern of the apertures may vary according to the nature of the non-woven material, the thickness of the cover, etc. However, it is preferred that the apertures generally comprise a substantially uniform pattern, e.g. spaced between 3 to 5mm apart, preferably about 4mm apart. The apertures may be substantially circular or elliptical and may have a diameter of from 0.5 to 2mm, preferably 1mm. However, other open patterned shapes such as diamonds will also perform a suitable function.

The length of the land areas connecting the apertures may play a role in providing the tampon with improved insertion characteristics. The optimum length of the land areas is dependent upon the type of absorbent material which is used for the absorbent core.

The primary benefits of the tampon of the invention are, *inter alia,* improved softness, low coefficient of friction and reduced fibre loss can still be maintained by selective choice of bi-component fibre, thickness of fibre content and pattern of aperture.

The covered tampon of the invention is advantageous in that, inter alia the tampon cover achieved provides one or more of the following benefits, e.g. the cover is softer, thicker, more breathable, is smoother (i.e. with a lower coefficient of friction) and has improved wicking properties into the core of the tampon..

## Claims

1. A tampon comprising a generally elongated absorbent core of absorbent material and an outer cover comprising a non-woven textile material formed from bi-component fibres which can be secured or bonded to the absorbent core.

2. A tampon according to claim 1 **characterised in that** the non-woven material is an apertured non-woven coverstock.

3. A tampon comprising a generally elongated core of absorbent material and an outer cover comprising an apertured bi-component polymeric fibrous coverstock thermally bonded to the absorbent core.

4. A tampon according to claim 1 where the fixing to the absorbent core is obtained through thermal bonding.

5. A tampon according to claims 1 or 3 **characterised in that** the non-woven coverstock comprises multi-component fibres.

6. A tampon according to claims 1 or 3 **characterised in that** the tampon is a digital tampon

7. A tampon according to claims 1 or 3 **characterised in that** the tampon is an applicator tampon.

8. A tampon according to claims 1 or 3 **characterised in that** the bi-component fibre of the coverstock comprises a first thermoplastic polymeric component.

9. A tampon according to claim 8 **characterised in that** the polymeric component is a polyolefin.

10. A tampon according to claim 9 **characterised in that** the polyolefin is selected from the group comprising polypropylene, polyethylene; polyolefin copolymers, such as ethylene-vinyl acetate ("EVA"), ethylene-propylene, ethylene-acrylates, and ethylene-acrylic acid and salts thereof; halogenated polymers; polyesters and polyester copolymers; polyamides and polyamide copolymers; polyurethanes and polyurethane copolymers; polystyrenes and polystyrene copolymers; and the like.

11. A tampon according to claim 10 **characterised in that** the polyolefin is polypropylene.

12. A tampon according to claim 8 **characterised in that** the fibre of the coverstock comprises a second thermoplastic polymeric component.

13. A tampon according to claim 12 **characterised in that** the second thermoplastic polymeric component is a polyolefin.

14. A tampon according to claim 13 **characterised in that** the polyolefin is selected from the group comprising polypropylene; polyolefin copolymers, such as ethylene-vinyl acetate ("EVA"), ethylene-propylene, ethylene-acrylates, and ethylene-acrylic acid and salts thereof; halogenated polymers; polyesters and polyester copolymers; polyamides and polyamide copolymers; polyurethanes and polyurethane copolymers; polystyrenes and polystyrene copolymers.

15. A tampon according to claim 14 **characterised in that** the second thermoplastic polymeric component is a polyethylene and/or its copolymers, especially linear low density polyethylene (LLDPE), low density polyethylene (LDPE) and EVA.

16. A tampon according to claims 1 or 3 **characterised in that** the coverstock fibre comprises a polyethylene (PE) sheath and polypropylene (PP) core.

17. A tampon according to claims 1 or 3 **characterised in that** the coverstock fibre comprises a polyethylene sheath and a polyester (PET) core.

18. A tampon according to claims 1 or 3 **characterised in that** the coverstock fibre comprises a polypropylene/polyethylene material.

19. A tampon according to claims 1 or 3 **characterised in that** the mass per unit area of the coverstock is from 10 to 30 gsm.

20. A tampon according to claim 19 **characterised in that** the mass per unit area of the coverstock is from 14 to 22 gsm.

21. A tampon according to claim 20 **characterised in that** the mass per unit area of the coverstock is from 16 to 20 gsm.

22. A tampon according to claim 21 **characterised in that** the mass per unit area of the coverstock is 18 gsm.

23. A tampon according to claims 1 or 3 **characterised in that** the coverstock is provided with a hydrophobic finish.

24. A tampon according to claims 1 or 3 **characterised in that** the coverstock is provided with a hydrophilic finish.

25. A tampon according to claims 1 or 3 **characterised in that** the caliper of the coverstock is less than 0.6 mm

26. A tampon according to claim 25 **characterised in that** the caliper of the coverstock is less than 0.5 mm.

27. A tampon according to claim 26 **characterised in that** the caliper of the coverstock is less than 0.45 mm.

28. A tampon according to claim 26 **characterised in that** the caliper of the coverstock is in the range of from 0.35 to 0.45 mm.

29. A tampon according to claim 28 **characterised in that** the caliper of the coverstock is from 0.4 to 0.45 mm.

30. A tampon according to claim 29 **characterised in that** the caliper of the coverstock is 0.41mm.

31. A tampon according to claims 1 or 3 **characterised in that** the tensile strength of the coverstock, is from 5 to 15 N/50mm (MD).

32. A tampon according to claim 31 **characterised in that** the tensile strength of the coverstock is from 8 to 12 N/50mm (MD).

33. A tampon according to claim 32 **characterised in that** the tensile strength of the coverstock is 10 N/50mm (MD).

34. A tampon according to claims 1 or 3 **characterised in that** the elongation at breaking point of the coverstock is less than 60%(MD).

35. A tampon according to claim 34 **characterised in that** the elongation at breaking point of the coverstock is less than 50%(MD).

36. A tampon according to claim 35 **characterised in that** the elongation at breaking point of the coverstock is less than 30%(MD).

37. A tampon according to claim 36 **characterised in that** the elongation at breaking point of the coverstock is less than 20%(MD).

38. A tampon according to claim 37 **characterised in that** the elongation at breaking point of the coverstock is 10%(MD).

39. A tampon according to claims 1 or 3 **characterised in that** the absorbent core comprising a material selected from the group comprising cellulosic materials, such as rayon, cotton, wood pulp, creped cellulose wadding, tissue wraps and laminates, peat moss, and chemically stiffened, modified, or cross-linked cellulosic fibres; synthetic materials, such as polyester fibres, polyolefin fibres, absorbent foams, absorbent sponges, superabsorbent polymers, absorbent gelling materials; formed fibres, such as capillary channel fibres and multilimbed fibres; combinations of materials, such as synthetic fibres and wood pulp including coformed fibrous structures.

40. A tampon according to claims 1 or 3 **characterised in that** an additive is included in the core and the additive is selected from the group comprising, antioxidants, UV absorbers, lubricants, antiblock and slip agents, plasticisers, nucleating agents, antistatic agents, flame retardants, pigments, dyes and inorganic or organic fillers.

41. A tampon according to claims 1 or 3 **characterised in that** an additive is included in the coverstock and the additive is selected from the group comprising, antioxidants, UV absorbers, lubricants, antiblock and slip agents, plasticisers, nucleating agents, antistatic agents, flame retardants, pigments, dyes and inorganic or organic fillers.

42. A tampon according to claims 1 or 3 **characterised in that** an additive is included in the coverstock and the additive is selected from the group comprising, surfactants, antimicrobials, zeolites and medicaments.

43. A tampon according to claims 2 or 3 **characterised in that** the apertures in the coverstock generally comprise a substantially uniform pattern.

44. A tampon according to claim 43 **characterised in that** the apertures are spaced from 3 to 5mm apart.

45. A tampon according to claim 44 **characterised in that** the apertures are spaced about 4mm apart.

46. A tampon according to claims 2 or 3 **characterised in that** the apertures are substantially circular in shape.

47. A tampon according to claims 2 or 3 **characterised in that** the apertures are substantially elliptical in shape.

48. A tampon according to claim 47 **characterised in that** the apertures have a diameter of from 0.5 to 2mm.

49. A tampon according to claim 48 **characterised in that** the apertures have a diameter of about 1mm.

50. A tampon substantially as described with reference to the accompanying examples.
